# EUROPEAN PATENT SPECIFICATION

(11) **EP 0 512 592 B1**
(45) Date of publication and mention of the grant of the patent: **09.07.1997**
(21) Application number: 92201012.9
(22) Date of filing: 08.04.1992
(51) Int. Cl.: A61B 1/00, A61F 9/00

(54) **Laser video endoscope**
Laservideoendoskop
Vidéo endoscope à laser

(30) Priority: 06.05.1991 US 696117; 29.07.1991 US 737054
(43) Date of publication of application: 11.11.1992
(73) Proprietor: Uram, Martin M.D., Little Silver, NJ 07739 (US)
(72) Inventor: Uram, Martin M.D., Little Silver, NJ 07739 (US)
(74) Representative: Berkenfeld, Helmut, Dipl.-Ing.

(56) References cited:
- EP-A- 0 305 170
- EP-A- 0 338 149
- EP-A- 0 428 446
- US-A- 4 607 622

## Description

### Reference To Related Applications

This invention relates in general to a small diameter endoscope used for medical purposes and more particularly to one in which illumination, viewing and laser operating functions are performed within a single relatively small diameter endoscope.

The endoscope of this invention is designed particularly for use in certain ophthalmological operations and thus the disclosure herein will relate to such an embodiment.

It is known to apply laser energy, and other types of energy, both directly and indirectly to various parts of the eye in order to effect surgery. For example, it is known to laser the peripheral retina for treatment of retina detachment. It is also known, in appropriate circumstances, to directly laser the ciliary processes as one of the treatments for glaucoma. Because of the difficulty of applying laser energy directly to the ciliary processes, the standard technique for disabling ciliary processes has been a cryogenic technique. This cryogenic technique involves applying a freezing probe on the external surface of the eye overlying the ciliary process on the inside of the eye. The ciliary processes are then frozen and thawed. This destroys the ciliary processes and thus reduces the aqueous output that builds up pressure. This process is fairly brutal in its immediate effect on the eye. Vision can be frequently lost. It is extremely difficult to titrate. There is a risk of shrinkage and atrophy of the eye due to overtreatment.

It is clearly preferable to apply a destructive element, such as a laser, directly to the ciliary processes. This has been done only where it is accompanied by a vitrectomy and lensectomy operation. That is only desirable or feasible in a very small number of cases.

It is a specific purpose of this invention to provide an intraocular endoscope that will be useful in photocoagulating any internal area of the eye including, most importantly, the pars plana region, the ciliary processes and the posterior aspect of the iris.

A further and related purpose of this invention is to allow more complete photocoagulation of the peripheral retina in the treatment of complicated retina detachment or proliferative retinopathies such as in diabetes mellitus.

It is a further and a related purpose of this invention to provide the above functions in a product which is relatively easy for a surgeon to use so that the operations involved can be precisely determined and can be more complete than is presently feasible.

Another related purpose of the invention is to provide an endoscope product that performs the above functions at a cost which makes it feasible for appropriate ocular surgery to be undertaken on a relatively widespread basis by a relatively large number of ophthalmologists.

An ophthalmic viewing instrument in the shape of a surgical endoscope including a hand piece and a probe at one end of the hand piece comprising a rigid tubular sidewall having a circular cross-section, an illumination guide comprising a first optical fiber extending longitudinally within said sidewall, an image guide comprising a second optical fiber extending longitudinally within said sidewall, and an optical laser guide comprising a third optical fiber extending longitudinally within said sidewall, said illumination guide, said image guide and said optical laser guide constituting substantially the only functional components within said tubular sidewall is known from EP 0 305 170 A2. In this known ophthalmic viewing instrument, the three optical fibers are solid elements and have the shape of rods. They extend parallel to each other, and the central points of these rods are positioned on an imaginary circle. This results into much free and unused area within the boundaries of the rigid tubular sidewall.

Also known in the prior art (US-A-4 607 622) is a fiber optic endoscope using bundles of optical fibers. These bundles of optical fibers function as observation and illuminating bundles.

The drawbacks of the prior art are overcome by the invention by a design being defined by the features of the characterising portion of claim 1.

By this design, the entire area within the tubular sidewall is efficiently and completely filled out since the diameters of the optical fibers are small in relation to the diameter of the tubular sidewall so that there are essentially no free unused areas intermediate the different optical fibers. For a given amount of function, i.e. the functions of illuminating, viewing and delivering laser energy, the endoscope according to the invention provides best results although the outer diameter is kept at a minimum. As mentioned above, the reason is that the illuminating fibers surround the image fibers and the laser fibers. For the purpose of an operation on the eye, the smallest possible diamter probe is highly advantageous. The surgical endoscope according to the invention may have an image guide that may have as many as 3,000 image fibers. Thus, each image is composed of 3,000 image pixels, each having approximately a three micron diameter.

The invention also provides advantageous further developments of the aforementioned basic idea. These further developments form the subject of several subclaims.

### Brief Description

In brief, this invention involves a fiber optic endoscope having a probe supported by a handpiece. The hand piece is connected through a relatively long flexible lead to a laser energy source, a source of illumination and an optical eye piece. The flexible lead, hand piece and probe all contain a laser optical fiber, an optical fiber image guide and an optical fiber illumination zone. The image guide fibers and the laser fiber are surrounded by the set of illumination fibers.

The laser fiber is a monofilament fiber that provides the required pulses of laser energy to effect operation. In one embodiment, it has a diameter of approximately 0.2 mm. The image guide is a set of high resolution fused quartz image fibers that provide a 3,000 pixel image, each pixel having a three micron diameter. The image guide has a diameter of 0.25 mm. An objective lens having a depth of field from down to about one mm is bonded to the distal end of the image guide.

This image guide and laser fiber are embedded within a set of fibers which carry illumination toward the distal end of the probe.

Light transmitted down the illumination fibers emerges at the distal end of the probe to provide illumination at the area of operation. The image of at least part of the area illuminated is transmitted back through the set of fibers that constitute the image guide to be viewed by the surgeon at an eyepiece or by video or still photography. With the image in view and the probe in position, the surgeon can then control the transmission of laser energy, typically pulses of laser energy, through the monofilament laser fiber to the zone of the operation.

### Brief Description Of The Figures

FIG. 1 is a mechanical schematic longitudinal view of an embodiment to this invention.

FIG. 2 is a cross-sectional view at the tip of the probe illustrating the relative deployment of the monofilament laser fiber 22, the 3,000 pixel image guide 24 and the multi-fiber illumination zone 26.

### Description Of The Preferred Embodiments

As shown in the FIGs., one embodiment of the endoscope of this invention has a hand piece 10 and a probe 12, which are connected through a first flexible cable 14 to a connector 16. An eyepiece 18 is optically coupled to the connector 16 for viewing purposes. A second flexible cable 20 extends out of the side of the connector 16.

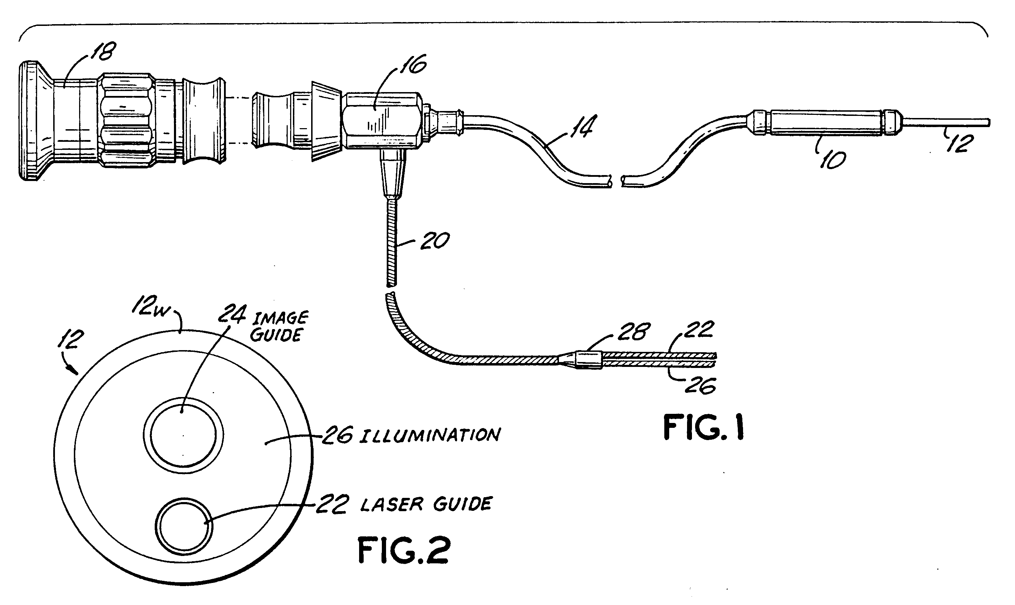

Within the probe 12, the hand piece 10 and the first flexible cable 14 there is deployed three separate sets of optical fibers that perform three separate functions. These are shown in the cross-sectional view of FIG. 2. This cross-sectional view is one taken at the tip of the probe 12. Within the probe 12 there is a monofilament laser fiber 22, an image guide 24 and an illumination zone 26. The laser fiber 22 is a monofilament optical fiber that delivers the laser energy at the tip of the probe 12 for performing operations. The image guide 24 is a set of high resolution fused quartz image fibers that provide a 3,000 pixel image, each pixel having a 3 micron diameter. The illumination zone 26 is composed of a large number of fibers which carry illumination toward the distal end of the probe 12. All of these optical fiber elements are quartz fibers.

In operation, light is transmitted down the illumination zone 22 to emerge at the distal end of the probe 12 to provide illumination at the area of operation. The image of at least part of the area illuminated is transmitted back through the image guide 24 to be viewed by the surgeon at the eyepiece 18. With the image in view and the probe 12 in position, the surgeon can then control the transmission of laser energy (usually pulses of laser energy) through the laser fiber 22 to the zone of the operation.

Because of the small diameter of the probe 12 (under one mm) this endoscope can be used for operations in areas (particularly for eye operations) where a combined viewing and operating endoscope was not hitherto possible.

At the juncture 16, the imaging set of optical fibers 16 is separated from the other two sets of optical fibers so that only the laser fiber 22 and illumination fibers 26 extend down through the tube 20. As indicated at the juncture 28, these two sets of fibers 22 and 26 are further separated to be appropriately connected to a source of laser energy for the optical fiber set 22 and to a source of light for the set of fibers that constitute the illumination zone 26.

It should be noted that the image provided by the image guide 24 can be applied to an eye piece 18 or can be displayed by a video or can be applied to create a still photograph. Indeed, it is anticipated that a video display might be preferable to facilitate the surgeon's positioning in order to manipulate the probe 12 properly.

In one embodiment that has been tested, the probe 12 has an outer diameter of 950 microns with a steel side wall 12w of 75 microns and thus an inner diameter of 800 microns.

In that embodiment, the laser fiber 22 is a monofilament fiber with an active diameter of 200 microns. The cladding and protective buffer layer to prevent mechanical abrasion of the cladding brings the diameter to 250 microns.

In that embodiment, the illumination zone 26 has 500 optical fibers, each fiber having a diameter of 30 microns including cladding. The optical fibers in the illumination zone 26 are strung randomly down the length of the instrument and are potted into place only at the tip of the operating probe 12.

In that embodiment, the image guide 24 has 3,000 quartz fibers, each only 3 microns in diameter including cladding. The fibers are fused to provide a single convenient to handle guide with 3,000 pixels. The guide wall is a thin black protective pvc sleeve 45 microns thick. This image guide 24 has a 250 micron diameter which with the PVC sleeve becomes 340 microns.

In that embodiment, the probe 12 is 30 mm long, the hand piece 10 in 40 mm long and the cable 14 is 410 mm long.

In one preferred embodiment, which has been tested, an objective lens is bonded to the distal end of the image guide 24 fibers and provides a depth of field from one mm to infinity with a field of view of 70 degrees. A depth of field that can go down to as little as one mm is important to aid the surgeon to position the end of the laser guide 22 as close as one mm from the tissue on which the laser energy is to be delivered. It is important to have the distal end of the laser guide and the distal end of the image guide at the same plane. The reason relates to a combination of the fact that (a) with a single probe there is no stereopsis, (b) the need to deliver the laser energy as close as possible to the tissue being worked on, and (c) the importance of avoiding tissue puncture or contact.

The lens is a triple lens of a known type. It is bonded to the image guide 24 prior to assembling the image guide 24 in the illumination zone so that the distal surface of the lens (2 mm which is one embodiment) is flush with the distal end of the probe.

Very minute working distances have to be traversed by means of the operator's hand movements. These minute working distances are in part required by the fact that the laser energy should be delivered only to a specific small zone of tissue which is to be operated on. This requires that the distal end of the laser guide be brought as close as possible to the tissue. A distance of one mm is desirable. But it is essential that the surgeon be able to view the tissue being operated on at the one mm distance in order to avoid having the probe contact, damage or puncture the tissue.

In known types of operations where an operating microscope is employed, an image is provided with a degree of stereopsis which aids the surgeon in moving toward the tissue. But with a probe that incorporates both imaging and laser delivery, stereopsis is not available and it is essential that the laser guide not extend past the image guide in order to make sure that tissue damage is avoided.

Having a wide field of view, such as 70 degrees, is useful to enable the surgeon to locate the various tissue zone areas which have to be operated on and to which energy has to be delivered.

As is known in the art, the particular laser guide optical material is selected as a function of the frequency of the laser light pulses which are to be transmitted by the guide. In the embodiment tested, the laser used is a diode laser composed of gallium-aluminum-arsenide semi-conducting crystals to provide a wave length of about 810 nano meters (in the range of 780 nm to 850 nm)

The joints 28 and 16 where the different components 22, 24 and 26 of the interior of the probe 12 are brought together are fabricated as junctions by standard techniques known in the art including the use of heat shrink tubing at the connector 28.

The embodiment has the straight probe 12 shown.
Applicant believes there may be advantage in a slight curvature to the probe 12 so that the tip of the probe is displaced two to three mm from the axis. This might provide advantage in use of more readily clearing the lens of the eye.

It should be noted that the combination of the illumination zone, the image guide and laser guide within a single probe provides a particularly compact probe for performing these three functions, which because it is compact and circular in cross section requires a minimal incision of the cornea while meeting the objectives of this invention including an ability to access areas such as the ciliary processes which otherwise require the use of multiple instruments.

## Claims

1. Surgical endoscope including a hand piece (10), a connecting cable attached to a proximal end thereof and a probe (12) at one end of the hand piece (10) comprising:
a rigid tubular sidewall (12w) having a circular cross-section,
an illumination guide (26) comprising a first optical means extending longitudinally within said sidewall (12w),
an image guide (24) comprising a second optical means extending longitudinally within said sidewall (12w),
an optical laser guide (22) comprising a third optical means extending longitudinally within said sidewall (12w),
said illumination guide (26), said image guide (24) and said optical laser guide (22) constituting substantially the only functional components within said tubular sidewall (12w),
said image guide and said optical laser guide terminating in substantially the same plane at the distal end of said rigid probe
characterized in that
said illumination guide optical means (26) comprises a substantial plurality of optical fibers, said image guide optical means (24) consists of a set of optical fibers, and said optical laser guide optical means (22) comprises a monofilament laser fiber,
said image guide fibers (24) and said optical laser guide monofilament fiber (22) are positioned within and surrounded by said substantial plurality of optical fibers constituting said illumination guide (26), and
said image guide optical fibers (24), said optical laser guide monofilament fiber (22) and said substantial plurality of optical fibers forming said illumination guide (26) substantially fill the cross-sectional area within said tubular sidewall (12w) of said probe (12).

2. Endoscope according to claim 1, characterized in that
said combined set of optical fibers have an outer diameter no greater than 800 microns,
said image guide (24) is circular in cross-section and has an outer diameter of essentially 250 microns,
said laser guide (22) including cladding has an outer diameter of essentially 250 microns.

3. Endoscope according to claim 1, characterized by
an objective lens bonded to the distal end of said image guide (24) to provide a depth of field ranging between infinity and one millimeter.

4. Endoscope according to claim 1, characterized by
a connecting cable (14) attached to the proximal end of the hand piece (10).

## Patentansprüche

1. Chirurgisches Endoskop mit einem Handstück (10) und einem an dessen körpernahem Ende befestigten Verbindungskabel und mit einem Meßkopf (12) an einem Ende des Handstücks (10) mit
einer Kreisquerschnitt aufweisenden steifen rohrförmigen Seitenwand (12w),
einer innerhalb der Seitenwand (12w) in Längsrichtung verlaufenden Beleuchtungsführung (26) aus einem ersten optischen Mittel,
einer innerhalb der Seitenwand (12w) in Längsrichtung verlaufenden Bildführung (24) aus einem zweiten optischen Mittel,
einer innerhalb der Seitenwand (12w) in Längsrichtung verlaufenden optischen Laserführung (22) aus einem dritten optischen Mittel,
wobei die Beleuchtungsführung (26), die Bildführung (24) und die optische Laserführung (22) innerhalb der rohrförmigen Seitenwand (12w) im wesentlichen die einzigen funktionellen Bauteile darstellen und
wobei die Bild- und die optische Laserführung am körperfernen Ende des steifen Meßkopfes in im wesentlichen der gleichen Ebene enden,
dadurch gekennzeichnet, daß
das die Beleuchtungsführung (26) bildende optische Mittel im wesentlichen aus einer großen Zahl von optischen Fasern besteht, das die Bildführung (24) bildende optische Mittel aus einem Satz von optischen Fasern besteht und das die optische Laserführung (22) bildende optische Mittel aus einer monofilen Laserfaser besteht,
die die Bildführung (24) bildenden optischen Fasern und die die Laserführung (22) bildende monofile optische Faser innerhalb der die Beleuchtungsführung (26) bildenden großen Zahl von optischen Fasern angeordnet und von diesen umschlossen sind und
die die Bildführung (24) bildenden optischen Fasern, die die Laserführung (22) bildende monofile optische Faser und die die Beleuchtungsführung (26) bildende große Zahl von optischen Fasern die Querschnittsfläche innerhalb der rohrförmigen Seitenwand (12w) des Meßkopfes (12) im wesentlichen ausfüllen.

2. Endoskop nach Anspruch 1, dadurch gekennzeichnet, daß
die Gesamtheit der optischen Fasern einen Außendurchmesser von nicht mehr als 800 Mikron aufweist,
die Bildführung (24) im Querschnitt kreisförmig ist und einen Außendurchmesser von im wesentlichen 250 Mikron aufweist,
die Laserführung (22) einschließlich eines Überzuges einen Außendurchmesser von im wesentlichen 250 Mikron aufweist.

3. Endoskop nach Anspruch 1, gekennzeichnet durch
eine am körperfernen Ende der Bildführung (24) angebrachte Objektivlinse zum Ausbilden einer Bildtiefe zwischen Unendlich und einem Millimeter.

4. Endoskop nach Anspruch 1, gekennzeichnet durch
ein am körpernahen Ende des Handstücks (10) befestigtes Verbindungskabel (14).

## Revendications

1. Endoscope chirurgical comportant une pièce à main (10) et un câble de connexion fixé à l'extrémité de cette première, qui est située proche du corps, ainsi qu'une tête de mesure (12) se trouvant à une extrémité de la pièce à main (10) comprenant
une paroi latérale (12w) tubulaire rigide présentant une section circulaire
un guide d'éclairage (26) consistant en un premier moyen optique et s'étendant dans la direction longitudinale à l'intérieur de la paroi latérale (12w),
un guide d'image (24) consistant en un deuxième moyen optique et s'étendant dans la direction longitudinale à l'intérieur de la paroi latérale (12w),
un guide-laser optique (22) consistant en un troisième moyen optique et s'étendant dans la direction longitudinale à l'intérieur de la paroi latérale (12w),
le guide d'éclairage (26), le guide d'image (24) ainsi que le guide-laser optique (22) constituant pour l'essentiel les seuls composants fonctionnnels à l'intérieur de ladite paroi latérale tubulaire (12w) et
ledit guide d'image et ledit guide-laser optique se terminant pour l'essentiel dans le même plan à l'extrémité de la tête de mesure rigide, qui est située loin du corps,
**caractérisé en ce que**
le moyen optique formant le guide d'éclairage (26) se compose pour l'essentiel d'un grand nombre de fibres optiques, en ce que le moyen optique formant le guide d'image (24) se compose d'un jeu de fibres optiques et en ce que le moyen optique formant le guide-laser optique (22) se compose d'une fibre laser monofilaire,
en ce que les fibres optiques formant le guide d'image (24) et la fibre optique monofilaire formant le guide-laser (22) sont disposées à l'intérieur du grand nombre de fibres optiques formant le guide d'éclairage (26) et sont entourées de celles-ci et
en ce que les fibres optiques formant le guide d'image (24), la fibre optique monofilaire formant le guide-laser (22) et le grand nombre de fibres optiques formant le guide d'éclairage (26) remplissent pour l'essentiel l'aire de la section à l'intérieur de la paroi latérale tubulaire (12w) de la tête de mesure (12).

2. Endoscope selon la revendication 1, caractérisé par le fait que
l'ensemble des fibres optiques présente un diamètre extérieur qui n'est pas plus grand que 800 microns,
le guide d'image (24) est, en coupe transversale, réalisé de façon circulaire et présente un diamètre extérieur de 250 microns pour l'essentiel,
le guide-laser (22) y compris un revêtement présente un diamètre extérieur de 250 microns pour l'essentiel.

3. Endoscope selon la revendication 1, caractérisé par
une lentille d'objectif disposée à l'extrémité du guide d'image (24), qui est située loin du corps, ladite lentille étant destinée à réaliser une profondeur d'image comprise entre l'infini et un millimètre.

4. Endoscope selon la revendication 1, caractérisé par
un câble de connexion (14) fixé à l'extrémité de la pièce à main (10), qui est située proche du corps.
